# EUROPEAN PATENT SPECIFICATION

(11) **EP 4 699 642 B1**
(45) Date of publication and mention of the grant of the patent: **09.09.2026**
(21) Application number: 25195109.1
(22) Date of filing: 11.08.2025
(51) Int. Cl.: A61N 1/04

(54) **WRIST-WORN MEDICAL DEVICE**
MEDIZINISCHES GERÄT ZUM TRAGEN AM HANDGELENK
DISPOSITIF MÉDICAL PORTÉ AU POIGNET

(30) Priority: 12.08.2024 CN 202411097330
(43) Date of publication of application: 25.02.2026
(73) Proprietor: Fasikl Incorporated, Dallas, TX 75206 (US); Hangzhou Fasikl Technology Co., Ltd, Hangzhou, Zhejiang 310000 (CN)
(72) Inventor: YE, Bing, Hangzhou City, 310000 (CN); WANG, Baitong, Hangzhou City, 310000 (CN); Nguyen, Jules Anh Tuan, DALLAS, 75206 (US); ZHANG, Zhen, DALLAS, 75206 (US)
(74) Representative: Chung, Hoi Kan

(56) References cited:
- AU-A1- 2022 324 555
- CN-U- 216 022 686
- US-A1- 2023 200 503
- US-A1- 2023 321 430

## Description

### TECHNICAL FIELD

The present disclosure relates to the technical field of wrist-worn medical devices, and particularly relates to a wrist-worn medical device.

### BACKGROUND

Electrical energy can be delivered transcutaneously with a nerve stimulation system via electrodes on the skin surface to stimulate peripheral nerves.

A wrist-worn nerve stimulator is a compact wearable electrical stimulation device, which can be worn on the wrist, and through a plurality of electrode plates closely attached to a peripheral edge of the wrist and according to certain rules, can stimulate a median nerve, a radial nerve and/or an ulnar nerve distributed in the hand to achieve a therapeutic effect. In a wearable medical device in the prior art, particularly in a wrist-worn device, the electrode plates are generally made of a metal material, and the metal electrode plates are hard, which causes poor comfort of contact with wrist skin when wearing. Generally, disposable conductive paste/solution is used to reduce the resistance of contact with the human skin to achieve a better therapeutic effect. To maintain the elasticity and flexibility of a rubber material, electrode plates made of conductive rubber generally have a too high self-resistance. However, due to poor affinity with human skin, a relatively high interface resistance is generated, such that patients will have a strong prickling sensation during use, and even psychologically refuse to receive, thus ultimately affecting therapeutic effect.

A Chinese patent with the Application No. CN202310595835.5 provides a wet electrode strap with a plurality of hydrogel electrode plates arranged on elongated non-woven fabrics. Through various model configurations, wrist nerves can be precisely located after wearing. Further, hydrogel electrodes bring about safety and comfort, and are capable of reducing the prickling sensation. However, during daily activities, particularly during intense activities such as climbing, running, rope skipping and the like, hands will undergo a large range of motion, which can cause position offset of a connection strap and an electrode wristband. When the offset position is large, skin will be directly exposed to conductive sheets, which can cause the prickling sensation, such that patients even psychologically refuse to receive, thus ultimately affecting the therapeutic effect. If the connection strap is tied to the wrist too tightly to prevent the position offset, it will cause poor blood circulation and comfort, and affect health. Therefore, there is an urgent need for a wrist-worn medical device that is capable of maintaining good conducting stability during exercise and reducing the relative offset between the connection strap and the electrode wristband.

US 2023/321430 A1 represents further background art.

### SUMMARY

The present invention provides a wrist-worn medical device according to claim 1. Further aspects and preferred embodiments are defined in the dependent claims.

**In** order to prevent separation between conductive sheets and conductive sponges caused by the offset of a connection strap, so as to ensure that a wrist-worn medical device is capable of maintaining good conducting stability, the present disclosure provides a wrist-worn medical device.

The following technical solution is adopted for the wrist-worn medical device provided by the present disclosure:

A wrist-worn medical device, including:
an electrode strap, provided with conductive sponges, where the conductive sponges are configured for abutting against and conducting with conductive sheets;
a connection strap, provided with the conductive sheets, where the connection strap is further electrically connected to a host, the conductive sheets are electrically connected to the host, and the host and the conductive sheets are respectively arranged on both sides of the connection strap along its thickness direction; and
a limiting member, arranged on the connection strap, where when the connection strap abuts against the electrode strap, both the conductive sponges and the conductive sheets are located inside the limiting member.

After the electrode strap is fixed on a wrist through hydrogel, the connection strap and the host are worn on the wrist, such that the connection strap is aligned with and covers the electrode strap, and the conductive sponges are partially overlapped with and abut against the conductive sheets. The limiting member of the connection strap is just aligned with the conductive sponges and surrounds the conductive sponges, such that the conductive sheets and the conductive sponges are both located inside the limiting member, and the conductive sheets are limited from sliding to a position where the conductive sheets and the conductive sponges are staggered, which helps to improve the stability of covering the electrode strap after the connection strap is worn, and makes it less likely for the conductive sheets and the conductive sponges to slide relative to each other, thereby improving the stability of conductivity between the conductive sheets and the conductive sponges.

Preferably, the limiting member is arranged to be an annular structure protruding on a surface of the connection strap close to the electrode strap, and the conductive sheets are arranged inside the limiting member; one limiting member is configured; when the connection strap is fitted with the electrode strap, the conductive sponges are located inside the annular structure of the limiting member, and a spacing is reserved between an inner circumference of the limiting member and the conductive sponges and between the inner circumference of the limiting member and the conductive sheets; and during displacement of the conductive sheets inside the limiting member, the conductive sheets and the conductive sponges are always in an abutment state.

The limiting member protruding on the surface of the connection strap surrounds all the conductive sponges and the conductive sheets, and arrangement of the spacing between the limiting member and the conductive sheets and between the limiting member and the conductive sponges allows for the displacement of the conductive sheets to adjust a position of the connection strap, and ensures that the conductive sheets abut against and are conducted with the conductive sponges.

Preferably, each of the limiting members is arranged to be an annular structure protruding on a surface of the connection strap close to the electrode strap, and the conductive sheets are arranged inside the limiting members; a plurality of the limiting members are configured, and the number of the limiting members corresponds to the number of the conductive sponges; when the connection strap is fitted with the electrode strap, the conductive sponges and the conductive sheets are located inside the annular structures of the limiting members in one-to-one correspondence, and a spacing is reserved between an inner circumference of each of the limiting members and the conductive sponges and between the inner circumference of each of the limiting members and the conductive sheets; and during displacement of the conductive sheets inside the limiting members, the conductive sheets and the conductive sponges are always in an abutment state.

The limiting members protruding on the surface of the connection strap surround all the conductive sponges and the conductive sheets, and arrangement of the spacing between the limiting members and the conductive sheets and between the limiting members and the conductive sponges allows for the displacement of the conductive sheets to adjust a position of the connection strap. The number of the limiting members corresponds to the numbers of the conductive sheets and the conductive sponges, such that the conductive sheets and the conductive sponges are limited by the limiting members in a more precise manner, thus ensuring that the conductive sheets abut against and are conducted with the conductive sponges.

Preferably, each of the limiting members is a limiting ring, the limiting ring is rectangular, a length direction of the limiting ring is consistent with the length direction of the electrode strap, and the limiting ring surrounds all the conductive sheets.

Preferably, each of the limiting members is a limiting ring, the limiting rings are square, and a plurality of the limiting rings corresponding to the conductive sheets are arranged.

Preferably, a thickness of each of the conductive sponges is greater than the thickness of the limiting ring.

Preferably, a length of each of the conductive sheets is greater than a width of one corresponding conductive sponge, the width of each of the conductive sheets is less than the length of each of the conductive sponges, the conductive sheets are arranged to be perpendicular to the conductive sponges, and the conductive sheets and the conductive sponges are overlapped in centers thereof and form a "cross" shape.

Preferably, the arrangement of the conductive sheets and the conductive sponges in the length and the width provides a range for the displacement of the conductive sheets and the conductive sponges. When the conductive sheets are displaced, the conductive sheets keep abutment with the conductive sponges, thus further improving the stability of conductivity between the conductive sheets and the conductive sponges.

Preferably, an area of each of the conductive sheets is greater than an area of one corresponding conductive sponge; each of the limiting members includes a limiting groove, and the limiting groove is recessed in a middle of one of the conductive sheets; and when the connection strap is just aligned with and abuts against the electrode strap, the conductive sponge is located inside the limiting groove of the corresponding conductive sheet, and a spacing is reserved between a peripheral wall of one conductive sponge and an inner side wall of the corresponding limiting groove.

The arrangement of the conductive sponges in the thickness and the arrangement of the limiting grooves in the conductive sheets, limit the sliding of the conductive sheets relative to the conductive sponges, which helps to reduce processing troubles caused by external structures and simplify an internal structure of the wrist-worn medical device.

Preferably, a ribbon is arranged at a tail end of the connection strap, a hook-and-loop fastener sub-patch is arranged on the ribbon, and a hook-and-loop fastener mother patch is arranged on an outer surface of a middle section of the connection strap; and one end of the connection strap is fixedly connected to one side of the host, and a through hole for the other end of the connection strap to pass through is formed on the other side of the host.

After the electrode strap is fixed on the wrist, one end of the connection strap passes through the through hole, and then the connection strap is bent, such that the hook-and-loop fastener sub-patch on the connection strap is just aligned with the hook-and-loop fastener mother patch. Then the hook-and-loop fastener sub-patch and the hook-and-loop fastener mother patch are fitted with each other to fix the connection strap, such that the conductive sheets and the conductive sponges tightly abut against each other, which helps to improve the stability of conductivity between the conductive sheets and the conductive sponges.

Preferably, the thickness of each of the conductive sponges is greater than a depth of the limiting groove.

To sum up, the present disclosure has the following beneficial effects:

After the electrode strap is fixed on a wrist through hydrogel, the connection strap is worn on the wrist, such that the connection strap is aligned with and covers the electrode strap, and the conductive sponges are partially overlapped with and abut against the conductive sheets. The limiting member of the connection strap is just aligned with the conductive sponges and surrounds the conductive sponges, such that the conductive sheets and the conductive sponges are both located inside the limiting member, and the conductive sheets are limited from sliding to a position where the conductive sheets and the conductive sponges are staggered, which helps to improve the stability of covering the electrode strap after the connection strap is worn, and makes it less likely for the conductive sheets and the conductive sponges to slide relative to each other, thereby improving the stability of conductivity between the conductive sheets and the conductive sponges.

### BRIEF DESCRIPTION OF DRAWINGS

FIG. 1 is a structural schematic diagram of a wrist-worn medical device of the present disclosure when worn on a wrist.
FIG. 2 is a schematic diagram of an overall structure of a wrist-worn medical device in Embodiment 1 of the present disclosure.
FIG. 3 is a structural schematic diagram of Embodiment 3 of the present disclosure.

Reference numerals in the figures: 1, connection strap; 2, conductive sponge; 3, electrode strap; 4, conductive sheet; 5, host; 6, hydrogel; 7, limiting groove; 8, through hole; 9, wrist; 10, hook-and-loop fastener sub-patch; and 11, limiting member.

### DETAILED DESCRIPTION OF THE EMBODIMENTS

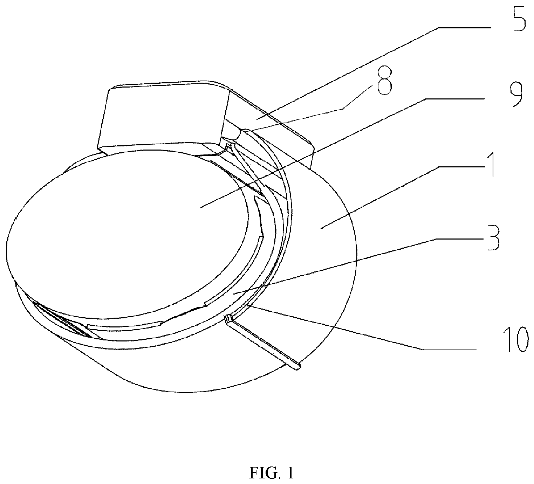

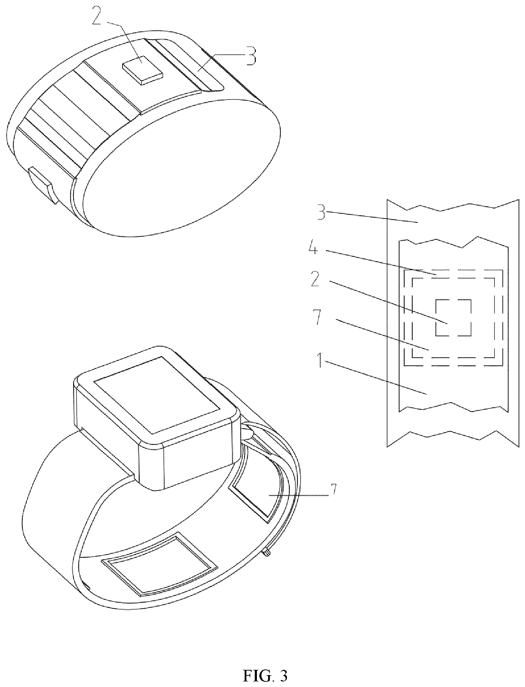

The present disclosure will be further described in detail below with reference to FIGs. 1-3.

The present disclosure discloses a wrist-worn medical device, which is detailed below in three embodiments of the present disclosure.

### Embodiment 1

A wrist-worn medical device, including:
an electrode strap 3, provided with conductive sponges 2, where the conductive sponges 2 are configured for abutting against and conducting with conductive sheets 4;
a connection strap 1, provided with the conductive sheets 4, where the connection strap 1 is further electrically connected to a host 5, the conductive sheets 4 are electrically connected to the host 5, and the host 5 and the conductive sheets 4 are respectively arranged on both sides of the connection strap 1 along its thickness direction; and
a limiting member 11, arranged on the connection strap 1, where when the connection strap 1 abuts against the electrode strap 3, both the conductive sponges 2 and the conductive sheets 4 are located inside the limiting member 11.

After the electrode strap 3 is fixed on a wrist 9 through hydrogel 6, the connection strap 1 is worn on the wrist 9, such that the connection strap 1 is aligned with and covers the electrode strap 3, and the conductive sponges 2 are partially overlapped with and abut against the conductive sheets 4. The limiting member 11 of the connection strap 1 is just aligned with the conductive sponges 2 and surrounds the conductive sponges 2, such that the conductive sheets 4 and the conductive sponges 2 are both located inside the limiting member 11, and the conductive sheets 4 are limited from sliding to a position where the conductive sheets 4 and the conductive sponges 2 are staggered, which helps to improve the stability of covering the electrode strap 3 after the connection strap 1 is worn, and makes it less likely for the conductive sheets 4 and the conductive sponges 2 to slide relative to each other, thereby improving the stability of conductivity between the conductive sheets 4 and the conductive sponges 2.

In this embodiment, a plurality of the conductive sheets 4 are arranged on the connection strap 1, and all the conductive sheets 4 are electrically communicated with the host 5 through interfaces of the connection strap 1.

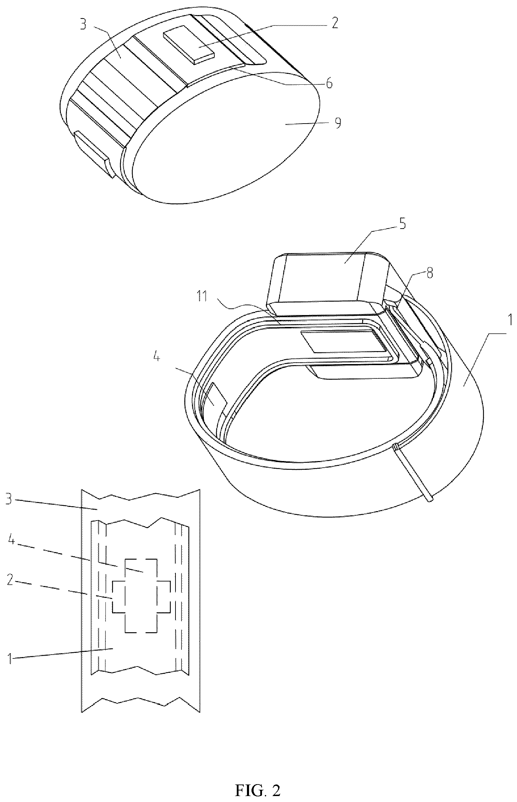

With reference to FIGs. 1 and 2, in this embodiment, the limiting member 11 is arranged to be an annular structure protruding on a surface of the connection strap 1 close to the electrode strap 3, and the conductive sheets 4 are arranged inside the limiting member 11. One limiting member 11 is configured. When the connection strap 1 is fitted with the electrode strap 3, the conductive sponges 2 are located inside the annular structure of the limiting member 11, and a spacing is reserved between an inner circumference of the limiting member 11 and the conductive sponges 2 and between the inner circumference of the limiting member 11 and the conductive sheets 4; and during displacement of the conductive sheets 4 inside the limiting member 11, the conductive sheets 4 and the conductive sponges 2 are always in an abutment state.

The limiting member 11 protruding on the surface of the connection strap 1 surrounds all the conductive sponges 2 and the conductive sheets 4, and arrangement of the spacing between the limiting member 11 and the conductive sheets 4 and between the limiting member 11 and the conductive sponges 2 allows for the displacement of the conductive sheets 4 to adjust a position of the connection strap 1, and ensures that the conductive sheets 4 abut against and are conducted with the conductive sponges 2.

With reference to FIGs. 1 and 2, in this embodiment, the limiting member 11 is a rectangular limiting ring, a length direction of the limiting ring is consistent with the length direction of the electrode strap 3, and the limiting ring surrounds all the conductive sheets 4.

With reference to FIGs. 1 and 2, in this embodiment, a thickness of each of the conductive sponges 2 is greater than the thickness of the limiting ring.

With reference to FIGs. 1 and 2, in this embodiment, a length of each of the conductive sheets 4 is greater than a width of one corresponding conductive sponge 2, the width of each of the conductive sheets 4 is less than the length of each of the conductive sponges 2, the conductive sheets 4 are arranged to be perpendicular to the conductive sponges 2, and the conductive sheets 4 and the conductive sponges 2 are overlapped in centers thereof and form a "cross" shape.

The arrangement of the conductive sheets 4 and the conductive sponges 2 in the length and the width provides a range for the displacement of the conductive sheets 4 and the conductive sponges 2. When the conductive sheets 4 are displaced, the conductive sheets 4 keep abutment with the conductive sponges 2, thus further improving the stability of conductivity between the conductive sheets 4 and the conductive sponges 2.

With reference to FIGs. 1 and 2, in this embodiment, a ribbon is arranged at a tail end of the connection strap 1, a hook-and-loop fastener sub-patch 10 is arranged on the ribbon, and a hook-and-loop fastener mother patch is arranged on an outer surface of a middle section of the connection strap 1. One end of the connection strap 1 is fixedly connected to one side of the host 5, and a through hole 8 for the other end of the connection strap 1 to pass through is formed on the other side of the host 5.

After the electrode strap 3 is fixed on the wrist 9, one end of the connection strap 1 passes through the through hole 8, and then the connection strap 1 is bent, such that the hook-and-loop fastener sub-patch 10 on the connection strap 1 is just aligned with the hook-and-loop fastener mother patch. Then the hook-and-loop fastener sub-patch and the hook-and-loop fastener mother patch are fitted with each other to fix the connection strap 1, such that the conductive sheets 4 and the conductive sponges 2 tightly abut against each other, which helps to improve the stability of conductivity between the conductive sheets 4 and the conductive sponges 2.

With reference to FIGs. 1 and 2, in this embodiment, the width of the electrode strap 3 is greater than the width of the connection strap 1.

### Embodiment 2

Embodiment 2 differs from Embodiment 1 in a specific structure of the limiting member 11.

With reference to FIGs. 1 and 2, in this embodiment, each of the limiting members 11 is arranged to be an annular structure protruding on a surface of the connection strap 1 close to the electrode strap 3, and the conductive sheets 4 are arranged inside the limiting members 11. A plurality of the limiting members 11 are configured, and the number of the limiting members 11 corresponds to the number of the conductive sponges 2. When the connection strap 1 is fitted with the electrode strap 3, the conductive sponges 2 and the conductive sheets 4 are located inside the annular structures of the limiting members 11 in one-to-one correspondence, and a spacing is reserved between an inner circumference of each of the limiting members 11 and the conductive sponges 2 and between the inner circumference of each of the limiting members 11 and the conductive sheets 4; and during displacement of the conductive sheets 4 inside the limiting members 11, the conductive sheets 4 and the conductive sponges 2 are always in an abutment state.

The limiting members 11 protruding on the surface of the connection strap 1 surround all the conductive sponges 2 and the conductive sheets 4, and arrangement of the spacing between the limiting members 11 and the conductive sheets 4 and between the limiting members 11 and the conductive sponges 2 allows for the displacement of the conductive sheets 4 to adjust a position of the connection strap 1. The number of the limiting members 11 corresponds to the numbers of the conductive sheets 4 and the conductive sponges 2, such that the conductive sheets 4 and the conductive sponges 2 are limited by the limiting members 11 in a more precise manner, thus ensuring that the conductive sheets 4 abut against and are conducted with the conductive sponges 2.

With reference to FIGs. 1 and 2, in this embodiment, each of the limiting members 11 is a square limiting ring, and a plurality of the limiting rings corresponding to the conductive sheets 4 are arranged.

### Embodiment 3

Embodiment 3 differs from Embodiment 1 in a specific structure of the limiting member 11.

With reference to FIGs. 1 and 2, in this embodiment, an area of each of the conductive sheets 4 is greater than an area of one corresponding conductive sponge 2. Each of the limiting members 11 includes a limiting groove 7, and the limiting groove 7 is recessed in a middle of one of the conductive sheets 4. When the connection strap 1 is just aligned with and abuts against the electrode strap 3, the conductive sponge 2 is located inside the limiting groove 7 of the corresponding conductive sheet 4, and a spacing is reserved between a peripheral wall of one conductive sponge 2 and an inner side wall of the corresponding limiting groove 7.

The arrangement of the conductive sponges 2 in the thickness and the arrangement of the limiting grooves 7 in the conductive sheets 4, limit the sliding of the conductive sheets 4 relative to the conductive sponges 2, which helps to reduce processing troubles caused by external structures and simplify an internal structure of the wrist-worn medical device.

The above are preferred embodiments of the present disclosure and are not intended to limit the protection scope of the present disclosure. Therefore, any equivalent changes made based on the structure, shape and principles of the present disclosure shall fall within the protection scope of the present disclosure. The invention, however, is defined by the appended claims.

## Claims

1. A wrist-worn medical device, comprising:
an electrode strap (3), comprising conductive sponges (2), wherein the conductive sponges are configured for abutting against and electrically contacting conductive sheets (4);
a connection strap (1), comprising the conductive sheets, wherein the connection strap is further electrically connected to a host (5),
the conductive sheets are electrically connected to the host, and the host and the conductive sheets are respectively arranged on opposite sides of the connection strap along its thickness direction; and
a limiting member (11), being arranged on the connection strap and configured to accommodate the conductive sponges, such that,
when the connection strap abuts against the electrode strap, both the conductive sponges and the conductive sheets are located inside the limiting member.

2. The wrist-worn medical device according to claim 1, wherein the limiting member is arranged to be an annular structure protruding on a surface of the connection strap close to the electrode strap, and the conductive sheets are arranged inside the limiting member; one limiting member is configured; when the connection strap is fitted with the electrode strap, the conductive sponges are located inside the annular structure of the limiting member, and a spacing is reserved between an inner circumference of the limiting member and the conductive sponges and between the inner circumference of the limiting member and the conductive sheets; and during displacement of the conductive sheets inside the limiting member, the conductive sheets and the conductive sponges are always in an abutment state.

3. The wrist-worn medical device according to claim 1, wherein each of the limiting members is arranged to be an annular structure protruding on a surface of the connection strap close to the electrode strap, and the conductive sheets are arranged inside the limiting members; a plurality of the limiting members are configured, and the number of the limiting members corresponds to the number of the conductive sponges; when the connection strap is fitted with the electrode strap, the conductive sponges and the conductive sheets are located inside the annular structures of the limiting members in one-to-one correspondence, and a spacing is reserved between an inner circumference of each of the limiting members and the conductive sponges and between the inner circumference of each of the limiting members and the conductive sheets; and during displacement of the conductive sheets inside the limiting members, the conductive sheets and the conductive sponges are always in an abutment state.

4. The wrist-worn medical device according to claim 2, wherein the limiting member is a limiting ring, the limiting ring is rectangular, a length direction of the limiting ring is consistent with the length direction of the electrode strap, and the limiting ring surrounds all the conductive sheets.

5. The wrist-worn medical device according to claim 3, wherein each of the limiting members is a limiting ring, the limiting rings are square, and a plurality of the limiting rings corresponding to the conductive sheets are arranged.

6. The wrist-worn medical device according to claim 4, wherein a thickness of each of the conductive sponges is greater than the thickness of the limiting ring.

7. The wrist-worn medical device according to claim 4, wherein a length of each of the conductive sheets is greater than a width of one corresponding conductive sponge, the width of each of the conductive sheets is less than the length of each of the conductive sponges, the conductive sheets are arranged to be perpendicular to the conductive sponges, and the conductive sheets and the conductive sponges are overlapped in centers thereof and form a "cross" shape.

8. The wrist-worn medical device according to claim 1, wherein an area of each of the conductive sheets is greater than an area of one corresponding conductive sponge; each of the limiting members comprises a limiting groove, and the limiting groove is recessed in a middle of one of the conductive sheets; and when the connection strap is just aligned with and abuts against the electrode strap, the conductive sponge is located inside the limiting groove of the corresponding conductive sheet, and a spacing is reserved between a peripheral wall of one conductive sponge and an inner side wall of the corresponding limiting groove.

9. The wrist-worn medical device according to claim 8, wherein the thickness of each of the conductive sponges is greater than a depth of the limiting groove.

10. The wrist-worn medical device according to claim 1, wherein a ribbon is arranged at a tail end of the connection strap, a hook-and-loop fastener sub-patch is arranged on the ribbon, and a hook-and-loop fastener mother patch is arranged on an outer surface of a middle section of the connection strap; and one end of the connection strap is fixedly connected to one side of the host, and a through hole for the other end of the connection strap to pass through is formed on the other side of the host.

## Patentansprüche

1. Am Handgelenk tragbare medizinische Vorrichtung, umfassend:
ein Elektrodenband (3), umfassend leitfähige Schwämme (2), wobei die leitfähigen Schwämme dazu ausgebildet sind, an leitfähigen Folien (4) anzuliegen und mit diesen elektrisch zu kontaktieren;
ein Verbindungsband (1), umfassend die leitfähigen Folien, wobei das Verbindungsband ferner elektrisch mit einem Host (5) verbunden ist, die leitfähigen Folien elektrisch mit dem Host verbunden sind, und der Host und die leitfähigen Folien jeweils auf gegenüberliegenden Seiten des Verbindungsbandes entlang dessen Dickenrichtung angeordnet sind; und
ein Begrenzungselement (11), das an dem Verbindungsband angeordnet und dazu ausgebildet ist, die leitfähigen Schwämme aufzunehmen, derart, dass, wenn das Verbindungsband an dem Elektrodenband anliegt, sowohl die leitfähigen Schwämme als auch die leitfähigen Folien innerhalb des Begrenzungselements angeordnet sind.

2. Am Handgelenk tragbare medizinische Vorrichtung nach Anspruch 1, wobei das Begrenzungselement als eine auf einer dem Elektrodenband nahen Oberfläche des Verbindungsbandes vorstehende ringförmige Struktur ausgebildet ist und die leitfähigen Folien innerhalb des Begrenzungselements angeordnet sind; wobei ein Begrenzungselement vorgesehen ist; wobei, wenn das Verbindungsband mit dem Elektrodenband zusammengefügt ist, die leitfähigen Schwämme innerhalb der ringförmigen Struktur des Begrenzungselements angeordnet sind und ein Abstand zwischen einem Innenumfang des Begrenzungselements und den leitfähigen Schwämmen sowie zwischen dem Innenumfang des Begrenzungselements und den leitfähigen Folien vorgesehen ist; und wobei während des Verschiebens der leitfähigen Folien innerhalb des Begrenzungselements die leitfähigen Folien und die leitfähigen Schwämme stets aneinander anliegen.

3. Am Handgelenk tragbare medizinische Vorrichtung nach Anspruch 1, wobei jedes der Begrenzungselemente als eine auf einer dem Elektrodenband nahen Oberfläche des Verbindungsbandes vorstehende ringförmige Struktur ausgebildet ist und die leitfähigen Folien innerhalb der Begrenzungselemente angeordnet sind; wobei mehrere Begrenzungselemente vorgesehen sind und die Anzahl der Begrenzungselemente der Anzahl der leitfähigen Schwämme entspricht; wobei, wenn das Verbindungsband mit dem Elektrodenband zusammengefügt ist, die leitfähigen Schwämme und die leitfähigen Folien jeweils einander paarweise zugeordnet innerhalb der ringförmigen Strukturen der Begrenzungselemente angeordnet sind und ein Abstand zwischen einem Innenumfang jedes der Begrenzungselemente und den leitfähigen Schwämmen sowie zwischen dem Innenumfang jedes der Begrenzungselemente und den leitfähigen Folien vorgesehen ist; und wobei während einer Verschiebung der leitfähigen Folien innerhalb der Begrenzungselemente die leitfähigen Folien und die leitfähigen Schwämme stets aneinander anliegen.

4. Am Handgelenk tragbare medizinische Vorrichtung nach Anspruch 2, wobei das Begrenzungselement ein Begrenzungsring ist, der Begrenzungsring rechteckig ist, eine Längsrichtung des Begrenzungsrings mit der Längsrichtung des Elektrodenbandes übereinstimmt, und der Begrenzungsring alle leitfähigen Folien umgibt.

5. Am Handgelenk tragbare medizinische Vorrichtung nach Anspruch 3, wobei jedes der Begrenzungselemente ein Begrenzungsring ist, die Begrenzungsringe quadratisch sind, und mehrere den leitfähigen Folien zugeordnete Begrenzungsringe vorgesehen sind.

6. Am Handgelenk tragbare medizinische Vorrichtung nach Anspruch 4, wobei eine Dicke jedes der leitfähigen Schwämme größer ist als die Dicke des Begrenzungsrings.

7. Am Handgelenk tragbare medizinische Vorrichtung nach Anspruch 4, wobei eine Länge jeder der leitfähigen Folien größer ist als eine Breite eines zugehörigen leitfähigen Schwamms, die Breite jeder der leitfähigen Folien kleiner ist als die Länge jedes der leitfähigen Schwämme, die leitfähigen Folien senkrecht zu den leitfähigen Schwämmen angeordnet sind, und die leitfähigen Folien und die leitfähigen Schwämme sich in ihren Mittelpunkten überlappen und eine "Kreuz"-Form bilden.

8. Am Handgelenk tragbare medizinische Vorrichtung nach Anspruch 1, wobei eine Fläche jeder der leitfähigen Folien größer ist als eine Fläche eines zugehörigen leitfähigen Schwamms; jedes der Begrenzungselemente eine Begrenzungsnut umfasst, wobei die Begrenzungsnut in der Mitte einer der leitfähigen Folien ausgespart ist; und wobei, wenn das Verbindungsband exakt an dem Elektrodenband ausgerichtet ist und an diesem anliegt, der leitfähige Schwamm in der Begrenzungsnut der zugehörigen leitfähigen Folie angeordnet ist, und ein Abstand zwischen einer Umfangswand eines leitfähigen Schwamms und einer Innenseitenwand der zugehörigen Begrenzungsnut vorgesehen ist.

9. Am Handgelenk tragbare medizinische Vorrichtung nach Anspruch 8, wobei die Dicke jedes der leitfähigen Schwämme größer ist als eine Tiefe der Begrenzungsnut.

10. Am Handgelenk tragbare medizinische Vorrichtung nach Anspruch 1, wobei an einem Endabschnitt des Verbindungsbandes eine Lasche angeordnet ist, an der Lasche ein Klettverschluss-Unterteil angeordnet ist, und auf einer Außenfläche eines Mittelabschnitts des Verbindungsbandes ein Klettverschluss-Oberteil angeordnet ist; und wobei ein Ende des Verbindungsbandes fest mit einer Seite des Hosts verbunden ist und auf der anderen Seite des Hosts ein Durchgangsloch ausgebildet ist, durch welches das andere Ende des Verbindungsbandes hindurchführbar ist.

## Revendications

1. Dispositif médical porté au poignet, comprenant :
une bande d'électrode (3), comprenant des éponges conductrices (2), dans lequel les éponges conductrices sont configurées pour venir en butée contre des feuilles conductrices (4) et entrer en contact électrique avec celles-ci ;
une sangle de connexion (1), comprenant les feuilles conductrices, dans lequel la sangle de connexion est en outre connectée électriquement à un hôte (5), les feuilles conductrices sont connectées électriquement à l'hôte, et l'hôte et les feuilles conductrices sont respectivement disposés sur des côtés opposés de la sangle de connexion selon la direction de son épaisseur ; et
un élément de limitation (11), disposé sur la sangle de connexion et configuré pour recevoir les éponges conductrices, de telle sorte que, lorsque la sangle de connexion vient en butée contre la bande d'électrode, les éponges conductrices et les feuilles conductrices sont toutes deux situées à l'intérieur de l'élément de limitation.

2. Dispositif médical porté au poignet selon la revendication 1, **caractérisé en ce que** l'élément de limitation est agencé sous la forme d'une structure annulaire faisant saillie sur une surface de la sangle de connexion proche de la bande d'électrode, et les feuilles conductrices sont disposées à l'intérieur de l'élément de limitation ; un seul élément de limitation est disposé ; lorsque la sangle de connexion est ajustée avec la bande d'électrode, les éponges conductrices sont situées à l'intérieur de la structure annulaire de l'élément de limitation, et un espacement est ménagé entre une circonférence intérieure de l'élément de limitation et les éponges conductrices, ainsi qu'entre la circonférence intérieure de l'élément de limitation et les feuilles conductrices ; et, lors du déplacement des feuilles conductrices à l'intérieur de l'élément de limitation, les feuilles conductrices et les éponges conductrices sont toujours en état de butée mutuelle.

3. Dispositif médical porté au poignet selon la revendication 1, **caractérisé en ce que** chacun des éléments de limitation est agencé sous la forme d'une structure annulaire faisant saillie sur une surface de la sangle de connexion proche de la bande d'électrode, et les feuilles conductrices sont disposées à l'intérieur des éléments de limitation ; une pluralité d'éléments de limitation sont prévus, et le nombre d'éléments de limitation correspond au nombre d'éponges conductrices ; lorsque la sangle de connexion est ajustée avec la bande d'é lectrode, les éponges conductrices et les feuilles conductrices sont situées à l'intérieur des structures annulaires des éléments de limitation selon une correspondance biunivoque, et un espacement est ménagé entre une circonférence intérieure de chacun des éléments de limitation et les éponges conductrices, ainsi qu'entre la circonférence intérieure de chacun des éléments de limitation et les feuilles conductrices ; et, lors du déplacement des feuilles conductrices à l'intérieur des éléments de limitation, les feuilles conductrices et les éponges conductrices sont toujours en état de butée mutuelle.

4. Dispositif médical porté au poignet selon la revendication 2, **caractérisé en ce que** l'élément de limitation est un anneau de limitation, l'anneau de limitation est rectangulaire, une direction longitudinale de l'anneau de limitation coïncide avec la direction longitudinale de la bande d'électrode, et l'anneau de limitation entoure l'ensemble des feuilles conductrices.

5. Dispositif médical porté au poignet selon la revendication 3, **caractérisé en ce que** chacun des éléments de limitation est un anneau de limitation, les anneaux de limitation sont carrés, et une pluralité d'anneaux de limitation correspondant aux feuilles conductrices sont disposés.

6. Dispositif médical porté au poignet selon la revendication 4, **caractérisé en ce qu'**une épaisseur de chacune des éponges conductrices est supérieure à l'épaisseur de l'anneau de limitation.

7. Dispositif médical porté au poignet selon la revendication 4, **caractérisé en ce qu'**une longueur de chacune des feuilles conductrices est supérieure à une largeur d'une éponge conductrice correspondante, la largeur de chacune des feuilles conductrices est inférieure à la longueur de chacune des éponges conductrices, les feuilles conductrices sont disposées perpendiculairement aux éponges conductrices, et les feuilles conductrices et les éponges conductrices se chevauchent en leurs centres respectifs et forment une forme en « croix ».

8. Dispositif médical porté au poignet selon la revendication 1, **caractérisé en ce qu'**une surface de chacune des feuilles conductrices est supérieure à une surface d'une éponge conductrice correspondante ; chacun des éléments de limitation comprend une rainure de limitation, et la rainure de limitation est en retrait au milieu d'une des feuilles conductrices ; et, lorsque la sangle de connexion est alignée avec précision avec la bande d'électrode et vient en butée contre la bande d'électrode, l'éponge conductrice est située à l'intérieur de la rainure de limitation de la feuille conductrice correspondante, et un espacement est ménagé entre une paroi périphérique d'une éponge conductrice et une paroi latérale intérieure de la rainure de limitation correspondante.

9. Dispositif médical porté au poignet selon la revendication 8, **caractérisé en ce que** l'épaisseur de chacune des éponges conductrices est supérieure à une profondeur de la rainure de limitation.

10. Dispositif médical porté au poignet selon la revendication 1, **caractérisé en ce qu'**un ruban est disposé à une extrémité arrière de la sangle de connexion, une sous-pastille de fixation auto-agrippante est disposée sur le ruban, et une pastille mère de fixation auto-agrippante est disposée sur une surface extérieure d'une section médiane de la sangle de connexion ; et une extrémité de la sangle de connexion est fixée de manière solidaire à un côté de l'hôte, et un trou traversant destiné au passage de l'autre extrémité de la sangle de connexion est ménagé sur l'autre côté de l'hôte.
